Europäisches Patentamt

European Patent Office

Office européen des brevets

(11) Numéro de publication: **0 030 885**
**B1**

(12) **FASCICULE DE BREVET EUROPEEN**

(45) Date de publication du fascicule du brevet: **10.10.84**    (51) Int. Cl.³: **C 12 M 3/00**, C 12 N 5/00

(21) Numéro de dépôt: **80401734.1**

(22) Date de dépôt: **04.12.80**

(54) **Nouvelles microparticules, leur préparation et leurs applications en biologie, notamment à la culture de cellules diploides.**

(30) Priorité: **05.12.79 FR 7929910**

(43) Date de publication de la demande:
**24.06.81 Bulletin 81/25**

(45) Mention de la délivrance du brevet:
**10.10.84 Bulletin 84/41**

(84) Etats contractants désignés:
**BE CH DE GB LI NL SE**

(56) Documents cités:
**FR-A-2 153 443**
**FR-A-2 370 792**
**FR-A-2 419 321**

**NATURE, vol. 216, 7 octobre 1967, A.L. VAN WEZEL: "Growth of cell-strains and primary cells on micro-carriers in homogeneous culture", pages 64 et 65**
**CHEMICAL ABSTRACTS, vol. 91, no. 13, 24 septembre 1979, page 416, abrégé 105967c COLUMBUS OHIO (US), R.C. HUGHES et al.: "Molecular requirements for the adhesion and spreading of hamster fibroblasts"**

(73) Titulaire: **INSTITUT PASTEUR**
**25-28, rue du Docteur Roux**
**F-75724 Paris Cedex 15 (FR)**
(73) Titulaire: **INSTITUT DE TECHNOLOGIE DES SURFACES ACTIVES**
**B.P. 233**
**F-60206 Compiegne Cedex (FR)**

(72) Inventeur: **Berneman, Armand**
**11, rue de Crussol**
**F-75011 Paris (FR)**
Inventeur: **David, Alain**
**Rue de Rollet Cuise la Motte**
**F-60350 Berneuil s.Aisne (FR)**
Inventeur: **Horaud, Florian**
**2, rue Albert de Mun**
**F-92190 Meudon (FR)**
Inventeur: **Segard, Emile**
**Village du Parc Rimbertlieu Villers s/Coudun**
**F-60150 Thourotte (FR)**

(74) Mandataire: **Peaucelle, Chantal et al**
**Cabinet Plasseraud 84, rue d'Amsterdam**
**F-75009 Paris (FR)**

Courier Press, Leamington Spa, England.

**0 030 885**

(56) Documents cités:
CHEMICAL ABSTRACTS, vol. 91, no. 7, 13
aou7t 1979, page 433, abrégé 53616r,
COLUMBUS OHIO (US), F. GRINNELL et al.:
"Initial adhesion of human fibroblasts in serum-
free medium: possible role of secreted
fibronectin"
CHEMICAL ABSTRACTS, vol. 88, no. 11, 13
mars 1978, page 314, abrégé 72157w,
COLUMBUS OHIO (US), M. HOOK et al.:
"Cold-insoluble globulin mediates the adhesion
of rat liver cells to plastic petri dishes"
EXPERIENTIA, vol. 36, no. 5, 15 mai 1980, F.
GRINNEL: "Fibroblast cell-substratum
interactions: role of cold insoluble globulin
(plasma fibronectin)", pages 505-507

## Description

L'invention a pour objet de nouvelles microparticules, leur préparation et leurs applications en biologie, notamment à la culture de cellules diploïdes animales et en particulier humaines, normales ou infectées, par exemple, par un virus ou un parasite.

On sait que les cellules diploïdes normales, qui sont des cellules d'origine embryonnaire, sont utilisées pour l'élaboration de divers produits d'origine cellulaire, tels les interférons, ou encore pour la culture de virus appropriés à la fabrication de vaccins viraux à usage humain.

Mais la production de ces éléments cellulaires à l'échelle industrielle se heurte aux exigences de culture des cellules diploïdes normales. En effet, contrairement aux cellules transformées, qui sont capables de se développer en suspension dans un milieu liquide, donc avec un rendement satisfaisant, les cellules diploïdes normales ne peuvent se multiplier que sur support solide.

Or, la culture en monocouche sur support solide d'une grande quantité de cellules en boîtes de Roux ou en rollers nécessite un grand nombre de récipients, ce qui constitue un facteur limitant et s'avère inutilisable aux fins d'une exploitation commerciale. On a cherché à favoriser le développement de ces cultures cellulaires en ajoutant un facteur d'adhésion tel que la fibronectine dans le milieu de culture. Mais les méthodes proposées, réalisées sur des surfaces planes ne conduisent pas à des taux d'adhésion et des développements cellulaires importants (voir Chemical Abstracts, vol. 91, N° 13, résumé N° 105967c, vol. 91, N° 7, résumé N° 53616r et vol. 88, N° 11, résumé N° 72157w et les articles correspondants respectivement à ces résumés, ainsi que l'article de F. GRINNEL dans Experientia vol. 36, N° 5, 1980, p. 505—507).

On a alors recherché des supports solides permettant de réaliser des cultures adaptables aux conditions de culture en masse dans des cuves de fermentation.

Les microparticules ou perles, en général sous forme de microbilles de 40 à 200 µm de diamètre environ, ont retenu, à cet égard, plus particulièrement l'attention. Elles permettent, en effet, de réaliser des cultures sensiblement homogènes adaptables en principe aux conditions de culture en fermenteur. Elles présentent, en outre, l'avantage d'offrir une surface de culture importante, ce qui permet d'envisager une augmentation du rendement en cellules produites.

On a ainsi proposé comme microsupports de cultures cellulaires, des microparticules formées de DEAE-Sephadex (voir l'article de A. L. Van Wezel dans Nature, vol. 216, 1967, p. 61—65, la demande de brevet FR publiée sous le N° 2 370 792 au nom de Massachussets Institute of Technology et la demande de brebet FR publiée sous le N° 2 419 321 au nom de Merk).

Cependant, de telles microbilles ne permettent pas de résoudre de manière entièrement satisfaisante les problèmes particuliers d'adhérence, de multiplication, de toxicité, rencontrés dans le cas de la culture de cellules diploïdes humaines normales ou infectées.

D'autres types de microparticules ont été préparées à partir de protéines gélifiantes (voir demande de brevet FR publiée sous le n° 2 153 443). Mais ces particules ont été élaborées pour piéger des enzymes et ne permettent pas de résoudre les problèmes spécifiques des cultures cellulaires sur microparticules.

Or, les travaux effectués dans ce domaine par les inventeurs, les ont amenés à constater que des microsupports particuliers, notamment pour culture de cellules diploïdes permettant une adhérence et une croissance extrêmement satisfaisante des cellules à leurs surfaces, pouvaient être élaborés à partir de certains types de protéines utilisés pour la constitution au moins de leurs surfaces.

L'invention vise donc de nouvelles microparticules favorisant, grâce à leur composition et les propriétés qui en résultent, l'adhérence et la croissance de cellules diploïdes humaines.

Elle vise également un procédé de préparation de ces microparticules.

Selon un autre aspect, l'invention vise l'application de ces microparticules à la culture de cellules, plus particulièrement de cellules diploïdes humaines normales, et à la culture de cellules humaines diploïdes infectées ou en vue de leur infection in situ.

Les microparticules, selon l'invention, que l'on désignera, indifféremment, également par le terme microsupports ou encore microbilles, sont caractérisées en ce qu'elles sont constituées de particules dont du moins le surface est formée d'une protéine réticulée cette protéine étant choisie parmi celles capables de former un gel, c'est-à-dire une masse viscoélastique, donnant lieu, après réticulation, à une masse de type fibreux, réticulée en mailles.

Ces particules possèdent avantageusement une capacité de charge d'environ 0,5 à 1,8 méq/gr de particules sèches. De préférence, la capacité de charge de ces microbilles est de 0,9 à 1,6 méq/gr et avantageusement de 1 ± 0,3 méq/gr de particules sèches.

D'une manière remarquable, les microbilles répondant aux caractéristiques définies ci-dessus s'avèrent particulièrement efficaces comme microsupports de culture de cellules diploïdes humaines, et ce, même avec des cultures cellulaires âgées ayant déjà subi plusieurs passages, c'est-à-dire ayant donné lieu à plusieurs générations.

Dans la suite de la description, il sera plus particulièrement fait référence aux cellules diploïdes humaines, compte-tenu de l'intérêt tout particulier que revêt l'invention pour leur culture. Mais, il est clair que l'invention s'applique egalement avec avantage à des cultures de cellules diploïdes animales.

La protéine, présente au moins à la surface de ces microbilles, constitue un support précieux pour l'adhérence des cellules et leur multiplication.

Selon un mode préféré de réalisation de l'invention, la protéine entrant dans la composition des microbilles et constituant au moins leur surface est formée par de la gélatine.

On sait que la gélatine est un produit d'hydrolyse partielle des collagènes. Selon son mode de fabrication, on distingue la gélatine obtenue par voie acide, possédant un point isoélectrique pHi compris entre 7 et 9 et celle obtenue par voie alcaline de pHi entre 4,7 et 5.

Ces deux types de gélatine peuvent être utilisés dans le cadre de l'invention, le gélatine obtenue par voie alcaline présentant l'avantage, grâce à ses propriétés de tension superficielle, de s'étaler plus aisément en donnant lieu à une surface sensiblement homogène, mieux appropriée aux applications biologiques, comme microsupports, envisagées.

Dans un autre mode de réalisation de l'invention, la protéine entrant dans la constitution des microbilles est constituée par de la fibronectine. Il s'agit d'une protéine de type fibrinogène, ayant un poids moléculaire de 440.000 environ, présente dans le plasma et à la surface de diverses cellules. Cette protéine est décrite, notamment dans Proc. Natl. Acad. Sci. USA. Vol. 76, N° 6, p. 2644—2648. Juin 79, par S.A. SANTORO et L.W. Cunningham.

Conformément à l'invention, les protéines entrant dans la composition des microbilles sont réticulées. L'agent de réticulation est avantageusement choisi parmi les agents de réticulation classiques. On a plus spécialement recours à un agent multifonctionnel ayant au moins deux groupes aldéhyde, azo, acide sulfonique, fluoro activés par des groupes nitro, azide imine ou chloro réactifs reliés à des structures cycliques ayant une résonance appropriée.

En raison de son efficacité et de sa disponibilité, on met avantageusement en oeuvre du glutaraldéhyde.

En vue de l'utilisation des microparticules de l'invention comme microsupports de cultures cellulaires, pour obvier aux risques éventuels de toxicité, vis-à-vis des cellules, les groupes fonctionnels de l'agent de réticulation, non engagés dans la réaction de réticulation sont bloqués.

Le blocage des groupes fonctionnels en question est effectué avantageusement à l'aide de la protéine entrant dans la constitution des microbilles.

Selon une variante d'exécution de l'invention, les microbilles sont essentiellement constituées par la protéine réticulée, de préférence par de la gélatine réticulée telle qu'évoquée ci-dessus, ou encore par de la fibronectine réticulée.

Selon une autre variante d'exécution de l'invention, les microbilles renferment la protéine réticulée à leur surface, sous forme d'un revêtement.

De telles microbilles sont de préférence formées de particules comportant un revêtement de protéine réticulée fixé sur un noyau d'ancrage.

Un noyau approprié est formé d'un matériau portant des groupements chimiques capables de réagir avec les groupements fonctionnels de la protéine du revêtement en donnant lieu à une liaison et possédant une densité permettant le maintien des particules en suspension dans un milieu de culture.

Comme matériau préféré de ce type, on a recours à du dextrane réticulé tel que du Séphadex, substitué par des groupements aminés tertiaires ou quaternaires, dans une proportion telle que la capacité de charge de la particule avant revêtement soit de l'ordre de 0,5 à 1,8 méq/gr de Séphadex sec, de préférence de 0,9 à 1,6 et avantageusement de $1 \pm 0,3$ méq/gr de Séphadex sec.

On notera que la capacité de charge des particules recouvertes de protéine est sensiblement du même ordre de grandeur.

Les groupements aminés sont plus spécialement constitués par des groupes diéthylaminoéthyle (DEAE) ou diéthyl-2-hydroxy-propylaminoéthyle (QAE).

Pour la préparation des microsupports de l'invention, on forme une solution, avantageusement aqueuse, d'une protéine présentant les caractéristiques définies ci-dessus, en une concentration permettant la réalisation de particules présentant les propriétés souhaitées en vue de leurs applications biologiques.

Cette solution est alors traitée pour former des particules entièrement constituées par la protéine en question ou, selon une variante, comportant cette protéine sous forme d'un revêtement.

Pour élaborer les particules de protéine, on traite la solution de protéine de manière à former des particules, de préférence des gouttelettes de diamètre de l'ordre de 40 à 200 $\mu$m notamment en la pulvérisant dans un bain consistant, par exemple, en un mélange d'huile végétale, d'alcool n-butylique et de glutaraldéhyde des concentrations adéquates permettant la prise en masse des gouttelettes. On peut également faire s'écouler la solution de protéine par un capillaire.

Quant au revêtement de protéine, il est avantageusement obtenu par mise en contact de ladite solution de protéine avec une suspension de préférence aqueuse de particules ayant un diamètre de l'ordre de 40 à 200 $\mu$m formées d'un matériau capable de constituer un noyau d'ancrage pour le revêtement de protéine tel que défini ci-dessus. Un tel matériau à faible capacité de charge est avantageusement préparé selon la technique décrite dans le brevet US N° 1.777.970. Les conditions opératoires, notamment de température, de durée, de pH étant choisies selon les produits mis en oeuvre, le revêtement et le capacité de charge désirés.

L'expérience montre que l'on obtient un revêtement satisfaisant en opérant à un pH voisin de 6 et à une température supérieure à l'ambiante, avantageusement inférieure à 40°C, une durée de réaction appropriée étant alors de l'ordre de 24 heures. Pour favoriser le contact des particules avec la protéine on soumet avantageusement le mélange réactionnel à agitation. Les particules de protéine, ou recouvertes de protéine, obtenues sont lavées puis mises en contact avec une solution, également avantageusement aqueuse, de l'agent de réticulation.

Les conditions optimales de température, durée et concentration sont mises au point selon des opérations de routine afin d'obtenir les particules présentant les caractéristiques souhaitées en fonction de la protéine et de l'agent de réticulation mis en oeuvre.

Comme déjà indiqué, les fonctions libres de l'agent de réticulation sont bloquées.

Après l'étape de réticulation, on procède donc avantageusement au lavage des particules qu'on met alors en contact avec une solution de préférence aqueuse de la protéine de la microbille, et ce dans les conditions permettant la réalisation du blocage désiré.

L'examen au microscope électronique des microbilles ainsi obtenues montre qu'elles présentent une surface ayant l'aspect d'une résille.

Les travaux effectués sur ces microbilles ont montré que, d'une manière inattendue, elles permettent une adhérence et une multiplication extrêmement satisfaisantes des cellules diploïdes humaines normales.

Il est également possible d'infecter par un virus ou un parasite les cellules qui ont poussé sur les microbilles, ce qui permet la préparation de virus, ou de parasites.

Les microbilles de l'invention sont alors avantageusement mises à profit comme microsupports de cultures cellulaires, plus spécialement de cellules diploïdes humaines normales ou infectées.

L'invention vise donc également un procédé de culture de cellules diploïdes humaines comportant la mise en oeuvre des microbilles définies ci-dessus.

Dans un mode préféré de réalisation de ce procédé, on forme une suspension stérile des microbilles en question dans un milieu approprié à la culture et à la croissance des cellules diploïdes humaines normales. On ensemence ce milieu dans des conditions stériles avec les cellules diploïdes, puis on le soumet à incubation dans des conditions de température et de durée permettant une multiplication satisfaisante des cellules.

L'étape d'ensemencement est avantageusement réalisée en mettant en oeuvre les cellules diploïdes humaines à raison de $10^5$ cellules environ par ml de milieu de culture pour 1 à 2 mg de microbilles environ par ml de milieu de culture et de préférence pour 1,5 à 1,7 plus spécialement 1,6 mg environ de microbilles.

L'étape d'ensemencement est également avantageusement réalisée en mettant en oeuvre les cellules diploïdes humaines à raison de $2,5 \times 10^5$ cellules par ml de milieu de culture pour 4 mg de microbilles par ml de milieu de culture.

Le milieu de culture dans lequel les microbilles sont en suspension est de préférence porté à la température qui sera ensuite utilisée lors de l'étape d'incubation.

Durant cette étape d'incubation, il est important d'offrir aux cellules le maximum de la surface des billes. On procède alors avantageusement à une agitation du milieu ensemencé pour le rendre homogène. On le laisse ensuite sédimenter afin de permettre le contact de la masse cellulaire avec les microbilles en vue de l'adhérence des cellules. Enfin, on agite légèrement pour ventiler l'interface couche cellulaire-microbilles.

Un traitement préalable des microbilles utilisées en tant que microsupports dans le procédé de culture de l'invention permet de manière favorable une meilleure croissance cellulaire.

Un traitement approprié comprend au moins une étape de stérilisation des microbilles dans du tampon phosphate appelé "PBS". Ces microbilles stériles sont ensuite préincubées dans du milieu de culture complet auquel éventuellement est ajouté de l'hydrolysat de lactalbumine. Ce milieu de culture comprend tous les éléments nécessaires à la croissance des cellules et sera utilisé dans l'étape d'incubation effectuée après l'ensemencement.

Cette étape de préincubation est avantageusement réalisée à une température comprise entre l'ambiante et 40°C environ, de préférence à 37°C et, dans ces conditions, durant environ 24 heures, en présence d'hydrolysat de lactalbumine.

Il est également préférable de soumettre, avant la préincubation, les microparticules à une étape de stérilisation. La stérilisation peut être effectuée en utilisant comme milieu de suspension le tampon de lavage, éventuellement additionné de la protéine entrant dans la constitution des microparticules.

Après, on rince les microparticules avec du PBS et on les préincube en milieu de culture Dulbecco contenant éventuellement de l'hydrolysat de lactalbumine, dans le cas où les microparticules sont ensemencées ultérieurement avec des cellules diploïdes humaines. Toutes ces opérations se font naturellement de manière stérile.

Les essais de croissance effectuées mettent en évidence les qualités précieuses des microsupports mis en oeuvre conformément à l'invention.

Comme le montrent les résultats rapportés dans les exemples qui suivent, leur efficacité apparaît remarquable aussi bien en ce qui concerne le taux d'adhérence des cellules que le nombre de dédoublements cellulaires.

On constate en effet avec intérêt que le pourcentage d'adhérence des cellules sur les microsupports par rapport au nombre initial de cellules ajoutées dans le flacon de culture est extrêmement satisfaisant et atteint des valeurs de l'ordre de 80%, dès 24 heures, après l'ensemencement.

Cette forte adhérence des cellules au début de la culture est avantageusement suivie d'une multiplication importante de ces cellules sur les microsupports.

Le nombre des dédoublements cellulaires observé après 5 à 7 jours de culture s'avère en effet supérieur à 2.

D'une manière avantageuse, les travaux effectués montrent de plus que les cellules ayant poussé sur les microsupports de l'invention peuvent être réutilisées dans un fermenteur de plus grand volume, ce qui montre la viabilité des cellules.

De tels résultats rendent possible l'utilisation du procédé de l'invention notamment pour la production à grande échelle de virus en vue de la préparation de vaccins viraux, ou d'autres produits cellulaires tels les interférons.

D'autres caractéristiques et avantages de l'invention apparaîtront dans les exemples qui suivent en se référant aux dessins sur lesquels:

— la figure 1 représente une photographie au microscope électronique d'une micro-particule recouverte d'un revêtement de gélatine, et

— la figure 2 représente la courbe de croissance d'une culture de cellules d'une souche MRC-5 sur des microsupports conformes à l'invention.

Exemple 1
Préparation de microbilles de Séphadex-DEAE recouvertes de gélatine réticulée

On prépare ces microbilles en mettant en oeuvre les trois étapes a) à c) suivantes, selon lesquelles:

a on procède au greffage des groupements de DEAE sur les microbilles de Séphadex,

b on recouvre les billes de Séphadex-DEAE obtenues avec de la gélatine,

c on procède à la réticulation de la gélatine et au blocage des groupes fonctionnels provenant de l'agent de réticulation.

Chacune de ces étapes est réalisée comme suit:

a) Greffage des groupements de DEAE sur les microbilles de Séphadex.

On laisse gonfler dans de l'eau distillée des microbilles de dextrane réticulé commercialisées par la Société PHARMACIA sous la marque Séphadex, du type G 50 médium, puis on les lave plusieurs fois avec de l'eau distillée. On forme ensuite une suspension de 50 g de ces microbilles dans 500 ml d'eau distillée. On prépare une solution aqueuse de 500 ml contenant 0,5 mole de chlorure de diéthylaminoéthyle et 0,75 mole de soude, un autre halogénure de DEAE, tel le bromure, et/ou un autre hydroxyde alcalin, telle la potasse, pouvant être utilisés. Le pH de cette solution est supérieur à 11. On ajoute cette solution à la suspension de microbilles dans un réacteur thermostaté à $60 \pm 1°C$. On soumet le mélange à agitation au sein du milieu avec une hélice à 4 pales en inox placées perpendiculairement. On laisse la réaction se poursuivre de 30 à 80 mn selon les essais effectués. On refroidit le mélange réactionnel par addition d'un volume d'eau distillée froide afin d'arrêter le greffage.

On laisse décanter et on enlève le surnageant. On répète plusieurs fois cette opération afin d'éliminer les réactifs, en particulier le DEAE, et d'obtenir un pH égal à 7.

On vérifie la capacité de charge des microbilles ainsi obtenues. On mesure à cet effet les anions chlorure échangés avec les microbilles. On lave tout d'abord les microbilles avec une solution d'acide chlorhydrique 0,1 N afin de saturer les sites d'échange par des ions $Cl^-$, puis on les lave avec une solution $10^{-4}$ N de HCl, pour enlever les ions chlorure non fixés et enfin avec une solution de sulfate de sodium à 10% en poids pour déplacer les anions chlorure par des anions sulfate. On recueille après ce lavage la solution qu'on titre avec une solution de nitrate d'argent 1M en utilisant du chromate de potassium comme indicateur.

Les mesures effectuées montrent que les microbilles obtenues possèdent une capacité de charge par gramme de Séphadex G 50 sec de 0,9 à 1,6 méq environ.

b) Recouvrement des microbilles avec de la gélatine.

On ajoute 100 ml de gélatine obtenue par voie alcaline telle que la gélatine d'os chaulée 240 Bis commercialisée par la société ROUSSELOT, à 10% en poids, 200 ml d'une suspension renfermant 10 g de microbilles telles qu'obtenues dans l'étape a).

Cette solution, de pH voisin de 7, est maintenue à 37°C pendant 24 heures avec une agitation faible. Au bout de 24 heures, les billes sont lavées plusieurs fois avec de l'eau distillée pour éliminer l'excès de gélatine.

c) Réticulation de la gélatine et bloquage des groupements fonctionnels provenant de l'agent de réticulation.

On remet les billes en suspension dans 200 ml d'eau distillée puis on ajoute 20 ml de glutaraldéhyde à 25% en solution aqueuse.

On laisse le mélange à température ambiante pendant 24 heures et sous faible agitation.

Au bout de 24 heures, on lave les billes plusieurs fois avec de l'eau distillée pour éliminer l'excès de glutaraldéhyde. On vérifie l'absence de réactif dans le surnageant de lavage en mesurant la densité optique à 280 nm (longueur d'onde d'adsorption du glutaraldéhyde).

Les fonctions libres du glutaraldéhyde sont ensuite bloquées avec de la gélatine.

On reprend alors les microbilles dans 200 ml d'eau distillée et on ajoute 100 ml de gélatine à 10%.

Cette solution est maintenue à 37°C pendant 10 heures environ. Pour éliminer l'excès de gélatine libre se trouvant dans le surnageant, les microbilles sont ensuite lavées (plusieurs fois) avec de l'eau distillée.

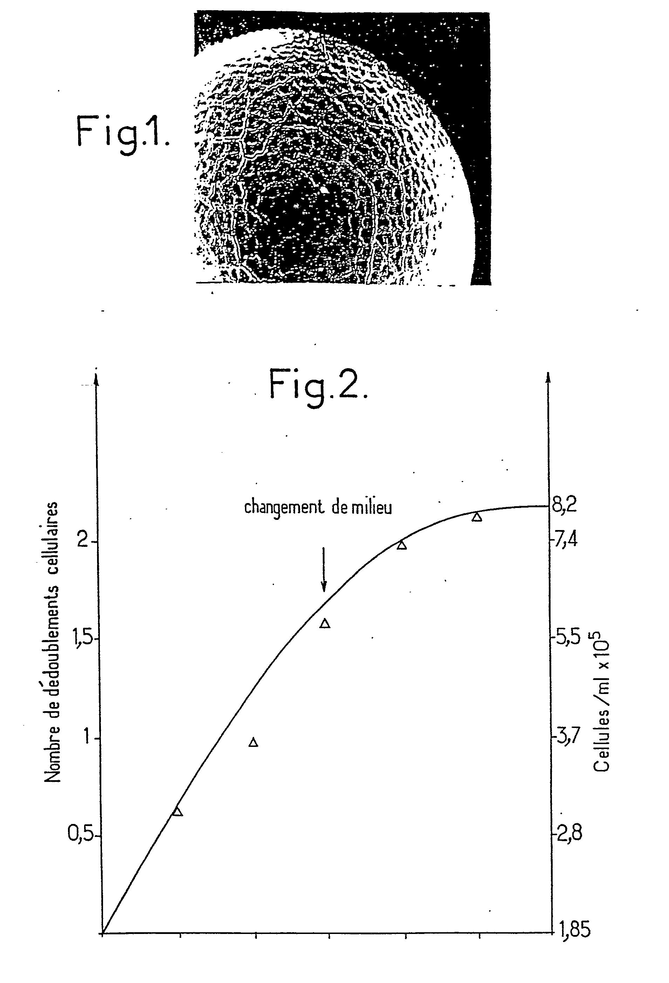

Sur la figure 1, on a rapporté une photographie en microscopie électronique de microbilles ainsi préparées. On constate que le revêtement de gélatine réticulée forme un fin filet à la surface des microbilles.

### Exemple 2
Traitement des microbilles obtenues dans l'exemple 1 en vue de leur application comme microsupports de cultures cellulaires

On lave tout d'abord les microbilles 5 ou 6 fois avec du PBS. On place ensuite une suspension de ces microbilles dans du PBS, contenant éventuellement 3 à 4% de gélatine dans un autoclave à 120°C pendant 20 mn, en vapeur humide.

### Exemple 3
Utilisation des microbilles traitées selon l'exemple 2 comme microsupports de culture cellulaire

Avant de procéder à l'étape d'ensemencement, on lave les microbilles traitées conformément à l'exemple 2 avec un milieu de culture appelé milieu Dulbecco, préparé en laboratoire, auquel on a jouté de l'hydrolysat de lactalbumine et le cas échéant des antibiotiques. Ce milieu Dulbecco est décrit notamment dans "International Association of Microbiological Societies Permanent Section of Microbiological Standardization", Minutes of the Seventh Meeting of the Committee on Cell Cultures, conférence qui s'est tenue à Genève le 14 Septembre 1970, édité par le Pr Hayflick et le Dr Perkins, p. 123—124, publié en 1971. Au troisième lavage, on incube la suspension de microbilles à 37°C durant 12 heures.

Au moment de l'essai, on prélève, après décantation, une fraction de suspension renfermant de 1,6 à 5 mg/ml et on les dépose stérilement dans un flacon du type spinner de 250 à 1000 ml. On laisse décanter les microbilles puis on les rince deux fois avec 10 ml de milieu Dulbecco auquel on a ajouté 10% de sérum aseptique de veau provenant de Medical and Veterinary Supplies, Slough, GG. Le volume est complété à 100 ml ou 150 ml ou 500 ml ou 1 litre, selon le volume du flacon.

On ensemence stérilement la suspension préchauffée à 37°C des cellules de poumon foetal humain MRC 5 obtenues du 27 au 32ème passage.

Les cellules proviennent d'un stock congelé préparé au laboratoire, répondant aux normes internationales. La culture de cellules est traitée par une solution dispersante de trypsine à 0,25%. Les cellules sont comptées et ajoutées dans le flacon de culture selon une proportion d'environ $10^5$ cellules pour 1,6 mg de microbilles par ml de suspension. Après l'ensemencement, on laisse le milieu de culture à 37°C pendant deux heures sous agitation, puis on met en marche le système d'agitation.

Afin de contrôler l'adhérence des cellules, le premier jour et la croissance les jours suivants, on prélève 1 ml de suspension de microbilles chaque jour.

On procède comme suit pour le comptage des cellules. La suspension prélevée est décantée puis rincée une fois avec un mélange de trypsine: versène volume pour volume (1,25‰: 0,1‰ concentrations finales). On les soumet ensuite à une incubation à 37°C en présence de 1 ml du mélange trypsine: versène.

Au bout de 5 à 10 mn, on soumet la suspension à agitation puis on ajoute du liquide isotonique et de même densité que les cellules. On attend la sédimentation des billes, puis on compte les cellules dans le surnageant avec un compteur Coulter commercialisé par Coultronics. Le pH du milieu est mesuré et ajusté régulièrement avec une solution de bicarbonate à 5,5%. On évalue la croissance cellulaire selon deux paramètres, à savoir:

1) le pourcentage d'adhérence des cellules sur les microsupports par rapport au nombre initial de cellules ajoutées dans le flacon de culture;

2) le nombre maximum de cellules obtenues après 5 à 7 jours de culture, ce qui permet de calculer le nombre de dédoublements cellulaires.

On obtient, 18 heures après le repiquage des cellules des pourcentages d'adhérence supérieurs en moyenne à 30—40% et pouvant atteindre 80 à 90%. Quant au nombre de dédoublements obtenu, il est généralement supérieur à 2 et peut atteindre, en opérant dans les conditions décrites ci-dessus, des valeurs voisines de 3.

L'allure des courbes de croissance des cellules sur les microbilles de l'invention montre l'apparition d'un plateau, qui représente la densité maximum des cellules, vers le sixième jour après l'ensemencement ou selon les essais le septième ou huitième jour.

On a représenté sur la figure 2 la courbe de croissance de cellules MRC-5 sur des microbilles de Séphadex G 50 DEAE recouvertes de gélatine, ayant une capacité de charge de 1,1 méq par gramme de Séphadex G 50 sec et non traité. L'essai de croissance a été effectué dans un volume de 100 ml avec 4 mg/ml de microbilles ayant subi le prétraitement décrit dans l'exemple 2, sans utilisation de gélatine et $2,5 \times 10^5$ cellules.

On a effectué un changement de milieu 4 jours après le début de l'essai afin d'éliminer les métabolites toujours rejetés dans le milieu et ainsi de maintenir la croissance. La courbe de croissance sur laquelle on a indiqué en abscisse la durée de l'essai, exprimée en jours, et en ordonnées le nombre de dédoublements cellulaires, présente l'aspect d'une courbe normale obtenue dans un système de culture en monocouche. On constate l'apparition d'un plateau vers le sixième jour correspondant à des dédoublements cellulaires supérieurs à 2.

Exemple Comparatif 3A

On procède à des essais comparatifs de croissance de cellules MRC-5 provenant du 28ème au 32ème passage sur des microbilles de l'invention d'une part et sur des microbilles de l'état de la technique non recouverte de gélatine réticulée.

Comme microbilles de l'état de la technique, on utilise des microbilles de type Séphadex G 50 DEAE (donc de dextrane réticulé sur lequel sont greffés des groupements de DEAE) possédant une capacité de charge de l'ordre de 1,5 méq/g de Séphadex G 50. A titre de comparaison, on utilise conformément à l'invention des microbilles de Séphadex G 50 DEAE recouvertes de gélatine réticulée préparées comme dans l'exemple 1, possédant une capacité de charge de 1,6 méq/g de Séphadex G 50 sec.

Dans ces essais comparatifs, les microbilles sont soumises à un prétraitement tel que décrit dans l'exemple 2, selon lequel on lave les microbilles avec du PBS puis on les place dans un autoclave, en milieu PBS.

L'étape d'ensemencement est réalisée comme dans l'exemple 3. On met en oeuvre les billes et les cellules selon des concentrations respectives de 4 mg/ml et de $2,5 \times 10^5$/ml de milieu de culture.

On observe une croissance pratiquement nulle avec les microbilles non revêtues d'une protéine réticulée et plus particulièrement de gélatine alors que le nombre de générations obtenu avec les microbilles de l'invention est égal à 2.

En ce qui concerne le taux d'adhérence, on constate qu'il est de l'ordre de 45 dans le cas de l'invention et voisin de 60 avec les microbilles de l'état de la technique.

Ces résultats mettent en évidence l'effet avantageux procuré par les microbilles de l'invention dont les caractéristiques propres permettent aux cellules MRC-5 utilisées dans le cadre de ces essais, qui sont des cellules âgées qui ont déjà subi un grand nombre de passages, d'adhérer fortement et de se multiplier.

Exemple 4

Etude de la croissance de cellules MRC-5 ayant poussé sur des microsupports de l'invention

On effectue plusieurs passages de cellules MRC-5 sur des microsupports de l'invention formés de Séphadex — DEAE de type G 50, recouvert de gélatine ayant une capacité de charge de 1,3 méq/g de Séphadex (avant l'ensemencement, les microsupports ont été lavés avec du PBS, puis incubés 30 mn dans du PBS renfermant 3% en poids de gélatine et enfin placés dans un autoclave à 120°C pendant 30 mn en présence de gélatine).

On détache ensuite les cellules, puis, dans un flacon de 100 ml, on les réensemence sur leur ancien support, les concentrations respectives étant de $2,8 \times 10^5$ cellules/ml de milieu de culture pour 5 mg/ml de microbilles.

On observe un taux d'adhérence de 95%, dix-huit heures après le repiquage des cellules et un nombre de dédoublement de 2,1.

Des essais analogues réalisés dans des flacons de 250 à 500 ml, avec respectivement $2,5 \times 10^5$ cellules/ml et $1,8 \times 10^5$ cellules/ml pour 5 mg/ml de microbilles conduisent à des taux d'adhérence de 98 et 61 et à des dédoublements cellulaires respectivement de 2 et 2,47.

Ces résultats montrent clairement que les cellules qui ont poussé sur ces microsupports de l'invention peuvent être réensemencés sur d'autres supports, ce qui est de grande importance pour le développement des cultures à grande échelle.

On notera également que la densité optimale obtenue en culture de 100 ml, qui est environ de $10^6$ cellules/ml est reproductible dans des flacons de culture de volume plus important de 250, 500 ou 1000 ml, ce qui montre la viabilité des microsupports de l'invention.

L'ensemble des résultats rapportés ci-dessus met en évidence que les microbilles de l'invention offrent des surfaces particulièrement appropriées à la culture des cellules diploïdes normales. Comme déjà indiqué, ces cellules, qui se trouvent sur les microsupports, peuvent être infectées par un virus ou un parasite cellulaire, ce qui permet l'élaboration de divers produits cellulaires, notamment de virus appropriés à la fabrication de vaccins viraux à usage humain.

La désignation "SEPHADEX" est une marque de fabrique pour du dextrane réticulé, commercialisée par Pharmacia Fine Chemicals Inc.

**Revendications**

1. Microparticules caractérisées en ce qu'elles sont constituées de particules ou perles dont au moins la surface est formée d'une protéine réticulée, choisie parmi celles capables de former un gel, c'est-à-dire une masse viscoélastique donnant lieu après réticulation à une masse de type fibreux, réticulée en mailles.

2. Microparticules selon la revendication 1, caractérisées en ce qu'elles possèdent à leur surface une capacité de charge d'environ 0,5 à 1,8 méq. par gramme de particules sèches.

3. Microparticules selon la revendication 2, caractérisées en ce qu'elles possèdent à leur surface une capacité de charge de 0,9 à 1,6 méq. et de préférence de $1 \pm 0,3$ méq. par gramme de particules sèches.

4. Microparticules selon l'une quelconque des revendications 1 à 3, caractérisées en ce que la protéine est de la gélatine, de préférence de la gélatine obtenue par voie alcaline.

5. Microparticules selon l'une quelconque des revendications 1 à 3, caractérisées en ce que la protéine est de la fibronectine.

6. Microparticules selon l'une quelconque des revendications 1 à 5, caractérisées en ce que la protéine est réticulée avec un agent multifonctionnel ayant au moins deux groupes aldéhyde, azo, acide sulfonique, fluoro activés par des groupes nitro, azide imine ou chloro réactifs reliés à des structures cycliques ayant une résonance appropriée.

7. Microparticules selon la revendication 6, caractérisées en ce que l'agent de réticulation est le glutaraldéhyde.

8. Microparticules selon les revendications 6 ou 7, caractérisées en ce que les groupements fonctionnels de l'agent de réticulation non engagés dans la réaction de réticulation sont bloqués, de préférence par la protéine entrant dans la constitution des microparticules.

9. Microparticules selon l'une quelconque des revendications 1 à 8, caractérisées en ce qu'elles sont constituées par la protéine réticulée.

10. Microparticules selon l'une quelconque des revendications 1 à 8, caractérisées en ce qu'elles renferment la protéine réticulée à leur surface, sous forme d'un revêtement.

11. Microparticules selon la revendication 10, caractérisées en ce que le revêtement de protéine réticulée est fixé sur un matériau constituant un noyau d'ancrage, comportant des groupements chimiques capables de réagir avec les groupements fonctionnels de la protéine du revêtement en donnant lieu à une liaison, et possédant une capacité de charge telle que définie dans les revendications 2 ou 3.

12. Microparticules selon la revendication 11, caractérisées en ce que le noyau d'ancrage est constitué par du dextrane réticulé substitué par des groupements aminés tertiaires ou quaternaires comme le groupement diéthylaminoéthyle (DEAE) ou diéthyl-2-hydroxy-propyl-aminoéthyle (QAE), dans une proportion telle que la capacité de charge de la microparticule corresponde aux valeurs données dans la revendication 2 ou 3.

13. Application des microparticules selon l'une quelconque des revendications 1 à 12, comme microsupports de culture cellulaire, plus spécialement pour la culture de cellules diploïdes animales ou humaines normales ou infectées par un virus ou un parasite cellulaire.

14. Procédé de culture de cellules diploïdes humaines, caractérisé en ce qu'on forme une suspension de microparticules selon l'une quelconque des revendications 1 à 11, dans un milieu approprié à la culture et à la croissance des cellules diploïdes, on ensemence cette suspension avec des cellules diploïdes humaines normales, on soumet le mélange à incubation dans des conditions de température et de durée permettant la multiplication cellulaire puis on recueille les cellules.

15. Procédé selon la revendication 14, caractérisé en ce qu'on met en oeuvre les cellules diploïdes à raison d'environ $10^5$ cellules par ml de milieu de culture pour environ 1 à 2 mg, de préférence environ 1,5 à 1,7 plus spécialement environ 1,6 mg de microbilles par ml de milieu de culture.

16. Procédé selon la revendication 14 ou 15, caractérisé en ce qu'on soumet préalablement les microbilles à un traitement comprenant au moins une étape d'incubation dans le milieu de culture, renfermant les éléments nécessaires à la croissance cellulaire, qui sera utilisé pour la culture, ce ou ces étapes de prêincubation étant avantageusement précédées d'au moins une étape de lavage des microbilles avec le tampon permettant d'éliminer les impuretés indésirables tout en n'altérant pas la surface des microbilles, puis une étape de stérilisation.

17. Procédé selon l'une quelconque des revendications 14 à 16, caractérisé en ce que les cellules diploïdes humaines normales cultivées sur les microsupports sont infectées à l'aide d'un virus ou de parasites se développant dans les cellules humaines aux fins de la préparation d'antigènes des dits virus ou desdits parasites.

**Patentansprüche**

1. Mikropartikel, dadurch gekennzeichnet, daß sie aus Teilchen oder Perlen bestehen, von denen mindestens die Oberfläche gebildet ist aus einem vernetzten Protein, ausgewählt aus den Proteinen, die fähig sind, ein Gel zu bilden, d. h. eine viskoelastische Masse, die zu einer Masse von faseriger Art, die in Schlingen vernetzt ist nach der Vernetzung führen.

2. Mikropartikel nach Anspruch 1, dadurch gekennzeichnet, daß sie an ihrer Oberfläche eine Ladungskapazität von ungefähr 0,5 bis 1,8 meq pro Gramm trockener Partikel.

3. Mikropartikel nach Anspruch 2, dadurch gekennzeichnet, daß sie an ihrer Oberfläche eine Ladungskapazität von 0.9 bis 1,6 meq besitzen und bevorzugt von $1 \pm 0,3$ meq pro Gramm trockener Teilchen.

4. Mikropartikel nach einem der Ansprüche 1 bis 3, dadurch gekennzeichnet, daß das Protein Gelatin ist, bevorzugt Gelatine, die auf alkalischem Wege erhalten wurde.

5. Mikropartikel nach einem der Ansprüche 1 bis 3, dadurch gekennzeichnet, daß das Protein Fibronektin ist.

6. Mikropartikel nach einem der Ansprüche 1 bis 5, dadurch gekennzeichnet, daß das Protein mit einem multifunktionellen Mittel vernetzt wird, das mindestens zwei Aldelhyd-, Azo-, Sulfonsäure-, oder Fluorgruppen aufweist, die durch reaktive Nitro-, Azid-, Imin- oder Chlorgruppen aktiviert sind, wobei die sogennante reaktive gruppen mit cyclischen Strukturen mit geeigneter Resonanz verbunden sind.

7. Mikropartikel nach Anspruch 6, dadurch gekennzeichnet, daß das Vernetzungsmittel Glutaraldehyd ist.

8. Mikropartikel nach Anspruch 6 oder 7, dadurch gekennzeichnet, daß die funktionellen Gruppen des Vernetzungsmittels, die nicht an der Vernetzungsreaktion beteiligt sind, blockiert sind, bevorzugt durch das Protein, das in die Konstitution der Mikropartikel eintritt.

9. Mikropartikel nach einem der Ansprüche 1 bis 8, dadurch gekennzeichnet, daß sie bestehen aus dem vernetzten Protein.

10. Mikropartikel nach einem der Ansprüche 1 bis 8, dadurch gekennzeichnet, daß sie an ihrer Oberfläche das vernetzte Protein tragen als Schicht.

11. Mikropartikel nach Anspruch 10, dadurch gekennzeichnet, daß die Schicht des vernetzten Proteins auf einem Material fixiert ist, das einen Kern als Verankerung bildet, der die chemischen Gruppen trägt, die fähig sind, mit den funktionellen Gruppen des Proteins der Schicht zu reagieren, wobei sie eine Bindung bewirken und die eine Ladungskapazität besitzen, wie sie in den Ansprüchen 2 oder 3 definiert ist.

12. Mikropartikel nach Anspruch 11, dadurch gekennzeichnet, daß der Verankerungskern gebildet wird aus vernetztem Dextran, das durch tert.-Amingruppen oder quaternäre Amingruppen, wie die Diethylaminoethylgruppe (DEAE) oder Diethyl-2-hydroxy-propyl-aminoethylgruppe (QAE) substituiert ist, in einem Verhältnis, daß die Ladungskapazität des Mikropartikels den Werten entspricht, die in Anspruch 2 oder 3 angegeben sind.

13. Anwendung der Mikropartikel nach einem der Ansprüche 1 bis 12 als Mikroträger der Zellkultur, insbesondere der Kultur der Zellen diploider Tiere oder Menschen, normale oder von einem Virus oder einem Parasiten befallene.

14. Verfahren zur Kultur von diploiden menschlichen Zellen, dadurch gekennzeichnet, daß man eine Suspension aus Mikropartikeln nach einem der Ansprüche 1 bis 11 bildet in einem Milieu, das für die Kultur und das Wachstum der diploiden Zellen geeignet ist, daß man diese Suspension mit den diploiden menschlichen normalen Zellen zuführt, daß man die Mischung einer Inkubation unterwirft, unter den Bedingungen der Temperatur und der Dauer, die die celluläre Vermehrung erlauben, und dann die Zellen sammelt.

15. Verfahren nach Anspruch 14, dadurch gekennzeichnet, daß man die diploiden Zellen zu ungefähr $10^5$ Zellen pro ml des Kulturmediums, für ungefähr 1 bis 2 mg, bevorzugt ungefähr 1,5 bis 1,7, insbesondere ungefähr 1,6 mg der Mikrokügelchen pro ml des Kulturmediums verwendet.

16. Verfahren nach Anspruch 14 oder 15, dadurch gekennzeichnet, daß man zuvor die Mikrokügelchen einer Behandlung unterwirft, die mindestens einen Inkubationsschritt in dem Kulturmedium umfaßt, das die notwendigen Elemente zum Zellwachstum enthält, die notwendig sein werden für die Kultur, wobei diesem Schritt oder diesen Schritten der Vorinkubation vorzugsweise mindestens ein Waschschritt der Mikrokügelchen vorausgeht mit dem Einsatz, der es erlaubt, die unerwünschten Unreinheiten zu entfernen, ohne die Oberfläche der Mikrokügelchen zu verändern, und dann ein Sterilisationsschritt.

17. Verfahren nach einem der Ansprüche 14 bis 16, dadurch gekennzeichnet, daß die diploiden menschlichen, normalen Zellen, die auf den Mikroträgern kultiviert sind, mit Hilfe eines Virus oder eines Parasiten infiziert sind, der sich in den menschlichen Zellen entwickelt zur Präparation von Antigenen dieser Viren oder dieser Parasiten.

## Claims

1. Microparticles characterized by the fact that they consist of particles or beads whose surface at least is formed from a cross-linked protein chosen from those capable of forming a gel, i.e. a viscoelastic mass giving rise after reticulation to a fibrous type mass reticulated in a mesh network.

2. Microparticles according to claim 1, having at their surface a charge capacity of about 0.5 to 1.8 meq per gram of dry particles.

3. Microparticles according to claim 2, having at their surface a charge capacity of 0.9 to 1.6 meq and preferably of $1 \pm 0.3$ meq per gram of dry particles.

4. Microparticles according to any one of claims 1 to 3, wherein the protein is gelatin, preferably gelatin obtained from an alkaline process.

5. Microparticles according to any one of claims 1 to 3, wherein protein is fibronectin.

6. Microparticles according to any one of claims 1 to 5, wherein the protein is cross-linked with a multifunction agent having at least two aldehyde, azo, sulfonic acid, fluoro groups activated by nitro, azide, imine chloro reactive groups connected with a cyclic structure having a suitable resonance.

7. Microparticles according to claim 6, wherein the cross-linking agent is glutaraldehyde.

8. Microparticles according to claim 6 or 7, wherein the functional groups of the cross-linking agent not engaged in the cross-linking reaction are blocked preferably by the protein which comprises said microparticles.

9. Microparticles according to any one of claims 1 to 8, wherein said microparticles consist of the cross-linked protein.

10. Microparticles according to any one of claims 1 to 8, wherein said particles are comprised of a surface coating of cross-linked protein.

11. Microparticles according to claim 10, wherein the cross-linked protein coating is fixed on a material forming an anchoring nucleus, comprising chemical groups capable of reacting with the functional groups of the protein of the coating to link therewith, and having a charge capacity such as defined in claims 2 or 3.

12. Microparticles according to claim 11, wherein the anchoring nucleus is formed of cross-linked dextran having tertiary or quaternary amino substituent groups such as diethylaminoethyl (DEAE) or diethyl-2-hydroxy-propylaminoethyl (QAE), in a proportion such that the charge capacity of the microparticles correspond to the values given in claim 2 or 3.

13. Application of the microparticles according to anyone of claims 1 to 12, as microcarriers for cellular culture, more especially for the culture of animal or human diploïd cells, normal or infected by a virus or a cellular parasite.

14. Process for the culture of human diploïd cells, comprising forming a suspension of microparticles according to any one of claims 1 to 11, in a medium suitable for the culture and the growth of the diploïd cells, seeding the suspension with normal human diploïd cells, subjecting the mixture to incubation under conditions of temperature and duration which allow cellular multiplication and then collecting the cells.

15. Process according to claim 14, wherein the diploïd cells are used at the rate of about $10^5$ cells per ml of culture medium for about 1 to 2 mg, preferably 1.5 to 1.7, more particularly about 1.6 mg of microbeads per ml of culture medium.

16. Process according to claim 14 to 15, wherein the microbeads are first submitted to a treatment comprising at least an incubation step in the culture medium, containing the ingredients necessary for the cellular growth, which will be used in the culture, said step or said steps of preincubation being advantageously preceded by at least a washing step of the microbeads with the buffer to eliminate the considerable impurities without adversely affecting the surface of the microparticles and then a sterilization step.

17. Process according to any one of claims 14 to 16, wherein the normal human diploïd cells cultivated on the microcarriers are infected by a virus or parasites which develop in the human cells for preparing antigens against said virus or said parasites.

# Fig.1.

# Fig.2.

changement de milieu

Nombre de dédoublements cellulaires

Cellules / ml x10⁵

8,2

7,4

5,5

3,7

2,8

1,85

2

1,5

1

0,5